# EUROPEAN PATENT APPLICATION

(11) **EP 1 886 628 A2**
(43) Date of publication of application: **13.02.2008**
(21) Application number: 07113323.5
(22) Date of filing: 27.07.2007
(51) Int. Cl.: A61B 5/07, H01Q 1/08

(54) **Antenna and body implant**

(30) Priority: 11.08.2006 GB 0615902
(71) Applicant: Zarlink Semiconductor Limited, Swindon, Wiltshire SN2 2QW (GB)
(72) Inventor: Wotherspoon, Tracy, Portskewett, NP26 5RT (GB)
(74) Representative: Robinson, John Stuart

(57) **Abstract**

A body implant antenna, comprises an electrically conductive electrode (2) formed on the surface of an enclosure (1) filled with a fluid (5) having a relatively permittivity greater than 10. The fluid is substantially electrically insulating and forms a substrate of the conductive electrode (2). Antenna size may therefore be reduced while permitting a relatively efficient antenna to be provided.

## Description

The present invention relates to a body implant antenna. Such an antenna may be used, for example, as part of a body implant for implanting in a human or other animal body. The invention also relates to a body implant comprising such an antenna.

Remote monitoring of medical or other implants may be achieved in various ways including radio frequency links. Existing US and European regulations have provided a dedicated communication band operating between 402 and 405 MHz. However, this poses a problem for antenna design in that simple quarter-wave and half-wave monopole or dipole antennae are physically impractical for use as implant antennae. For example, a conventional quarter-wave monopole antenna for operation at 403 MHz would normally have a length of 18.5cm, which is too long for most implant applications.

Conventional techniques have been used to provide antennae which are sufficiently small to be usable in implants. For example, antennae based on patches, loops and meandered dipoles have been used but create relatively inefficient antennae. Also, the physical length of an antenna may be reduced by forming antennae on high dielectric substrates. In particular, the effective wave length for antenna purposes is equal to a product of the wave speed and the frequency and the wave speed is inversely proportional to the square root of the permittivity of the substrate. However, substrate materials of high dielectric constant generally have relatively high losses and therefore provide poor antenna efficiency.

GB 2 390 545 discloses a hollow organ probe for providing treatment by heating an internal body cavity, such as the oesophagus. The heat source is a microwave antenna located inside a balloon but not touching the surface. The balloon contains a liquid which is heated by the microwave antenna so as to transfer heat to an internal body surface to perform therapy.

US 2004/0125019 discloses an antenna array with an operating band which can be varied in use. The array is formed on a hollow dielectric substrate with a facing ground plane. The resulting cavity can be filled with dielectric fluid whose composition can be actively varied by mixing different fluids within the cavity.

US 2005/0035916 discloses a reflecting antenna for steering an incident radio frequency beam. An array of discrete dielectric cells is formed on top of the conductive reflecting antenna. The amount of dielectric fluid in each cell is controlled so as to simulate different directions and shapes of the reflector.

According to a first aspect of the invention, there is provided a body implant antenna characterised by comprising an electrically conductive electrode provided with a substantially electrically insulating substrate comprising a fluid having a relative permittivity greater than 10.

The fluid may comprise a liquid. The liquid may comprise water. The water may be distilled and/or deionised.

The fluid may be of substantially fixed composition.

The fluid may be of substantially fixed mass.

The electrode may be formed on or in a surface of an enclosure containing the fluid. The electrode may be formed on an internal surface of the enclosure. The enclosure may be sealed. The surface may comprise a flexible membrane. The enclosure may have an external surface which is substantially inert to body fluids. The enclosure may be substantially impervious to the fluid and to body fluids.

The antenna may comprise an electronic circuit electrically connected to the electrode. The circuit may be disposed inside the enclosure. The circuit may be sealed inside a further enclosure. The fluid may be sealed between the enclosure and the further enclosure.

According to a second aspect of the invention, there is provided body implant comprising an antenna according to the first aspect of the invention.

It is thus possible to provide a relatively small antenna of relatively high efficiency. Such an antenna is suitable for use with implants.

The invention will be further described, by way of example, with reference to the accompanying drawings, in which:
Figure 1 is a diagram of an antenna constituting a first embodiment of the invention; and
Figure 2 is a diagram of an antenna constituting a second embodiment of the invention.

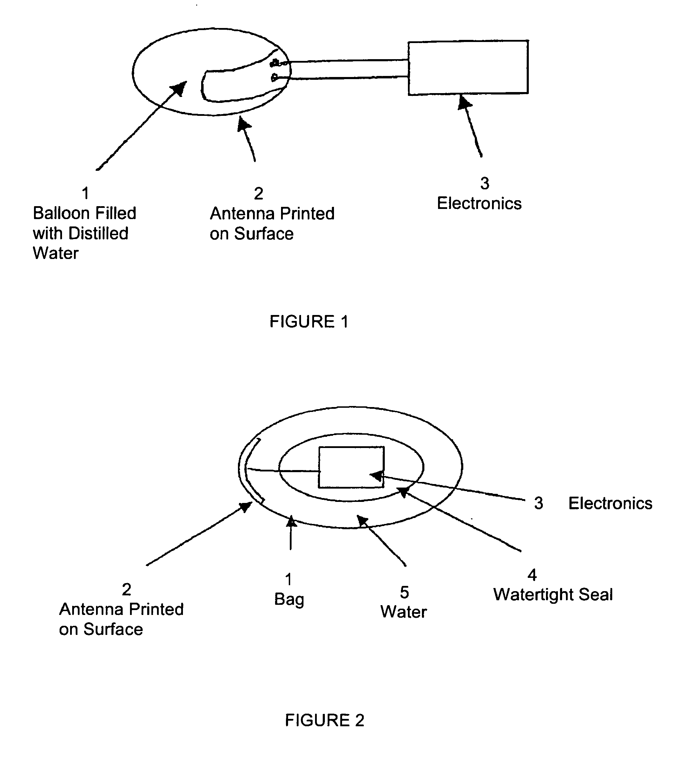

The antenna shown in Figure 1 comprises an enclosure in the form of a bag or "balloon" 1 on whose surface is printed an antenna electrode 2. The electrode 2 is illustrated as being formed on an external surface of the balloon 1 but may alternatively be formed on the internal surface of the balloon. The antenna is connected to an electronic circuit ("electronics") 3 for providing radio frequency communication between the electronic circuit 3 and a remote communication terminal.

The balloon 1 is filled with a fluid which forms an electrically insulating substrate for the antenna electrode 2. The fluid is of fixed composition and of fixed mass. The fluid comprises a liquid which is substantially electrically insulating and which has a relative permittivity greater than 10. In the specific example illustrated, the fluid is water but other liquids and gases may be used.

Distilled, fresh and sea water all have relative permittivities substantial equal to 81 and dielectric loss tangents substantial equal to zero. Relative permeabilities are substantially equal to 0.99 and magnetic loss tangents and magnetic saturation are substantially equal to zero. However, sea water has a conductivity of 4 S/m and fresh water has a conductivity of 0.01 S/m, which is generally unacceptably high and would result in excessive losses. Distilled water has a conductivity of 0.0002 S/m and is therefore very suitable for use as the fluid substrate of the antenna.

In general, the fluid is required to be cheap and to be inert and easily handled. In addition, when the antenna is to be used as a medical implant or for other medical purposes, the fluid must be substantially non-toxic. Distilled and/or deionised water are therefore suitable for such applications, although other fluids also fulfil these requirements.

When the antenna is to be used as an implant, the balloon 1 or other enclosure must be made of a material which is suitable for this purpose. For example, implants are frequently inserted into extra-cellular cavities within the human or other animal body. Such cavities often contain fluids which typically comprise mainly water but with relatively high ionic concentrations. In order to prevent the effect of osmosis removing water from the balloon 1 and to prevent diffusion from permitting ions to drift inside the balloon 1, the material of the balloon 1 must be biocompatible and impervious to water and to soda, potassium and chlorine ions and other body salts. It is also preferable for the conductor 2 not to be on the external surface of the balloon 1 so as to avoid the risk of electrolysis.

A type of material which may be used as the membrane forming the balloon 1 is known as stretch "synthetic skin" polymer and examples of this have been disclosed by Princeton University, New Jersey, USA at http://www.prism.princetou.edu/Sturm publications/JP.137.pdf. Gold conductors may be inlayed into the polymer to form the electrode 2 and may be stretched by up to 16% without change in resistance.

Another type of material which may be used as the membrane of the balloon 1 comprises anti-static or electromagnetic interference (EMI) shield bags. Such materials or bags comprise polythene and polyester coated with aluminium foil sandwiched between the layers and such materials are available in thickness from about 7 micrometres. Bags of this type and classified by military specifications MIL-B-81705C Type II class 1 and Type III class 1 are waterproof and are therefore suitable for this application.

Figure 2 illustrates an alternative arrangement in which the electronic circuit 3 is hermetically sealed within a water tight seal 4, which is disposed inside the balloon or bag 1. In this example, the antenna electrode 2 is printed on the interior surface of the bag 1 and is connected to the electronic circuit 3 by a connection passing through and sealed to the water tight seal 4 so as to preserve water tightness. The cavity between the water tight seal 4 and the bag 1 forms a sealed chamber containing the water 5 acting as the electrically insulating substrate. Such an arrangement may be more suitable for use as a medical or other implant as sharp edges may be avoided and the implant may be made semi-flexible and soft.

## Claims

1. A body implant antenna **characterised by** comprising an electrically conductive electrode (2) provided with a substantially electrically insulating substrate (1, 5) comprising a fluid (5) having a relative permittivity greater than 10.

2. An antenna as claimed in claim 1, **characterised in that** the fluid (5) comprises a liquid.

3. An antenna as claimed in claim 2, **characterised in that** the fluid (5) comprises water.

4. An antenna as claimed in claim 3, **characterised in that** the water (5) is distilled and/or deionised.

5. An antenna as claimed in any one of the preceding claims, **characterised in that** the fluid (5) is of substantially fixed composition.

6. An antenna as claimed in any one of the preceding claims, **characterised in that** the fluid (5) is of substantially fixed mass.

7. An antenna as claimed in any one of the preceding claims, **characterised in that** the electrode (2) is formed on or in a surface of an enclosure (1) containing the fluid.

8. An antenna as claimed in claim 7, **characterised in that** the electrode (2) is formed on an internal surface of the enclosure (1).

9. An antenna as claimed in claim 7 or 8, **characterised in that** the enclosure (1) is sealed.

10. An antenna as claimed in any one of claims 7 to 9, **characterised in that** the surface comprises a flexible membrane.

11. An antenna as claimed in any one of claims 7 to 10, **characterised in that** the enclosure (1) has an external surface which is substantially inert to body fluids.

12. An antenna as claimed in any one of claims 7 to 11, **characterised in that** the enclosure (1) is substantially impervious to the fluid and to body fluids.

13. An antenna as claimed in any one of the preceding claims, **characterised by** comprising an electronic circuit (3) electrically connected to the electrode (2).

14. An antenna as claimed in claim 13 when dependent on any one of claims 7 to 12, **characterised in that** the circuit (3) is disposed inside the enclosure (1).

15. An antenna as claimed in claim 14, **characterised in that** the circuit (3) is sealed inside a further enclosure (4).

16. An antenna as claimed in claim 15, **characterised in that** the fluid (5) is sealed between the enclosure (1) and the further enclosure (4).

17. A body implant comprising an antenna as claimed in any one of the preceding claims.
